(19) Europäisches Patentamt
European Patent Office
Office européen des brevets

(11) Publication number: **0 060 049**
**B1**

(12) **EUROPEAN PATENT SPECIFICATION**

(45) Date of publication of patent specification: **23.01.85**

(51) Int. Cl.[4]: **C 07 C 154/02**

(21) Application number: **82300900.6**

(22) Date of filing: **23.02.82**

(54) **Production of cyanoguanidine intermediates.**

(30) Priority: **10.03.81 GB 8107487**
**01.04.81 GB 8110259**
**05.05.81 GB 8113657**
**03.06.81 GB 8116948**
**29.06.81 GB 8120031**

(43) Date of publication of application:
**15.09.82 Bulletin 82/37**

(45) Publication of the grant of the patent:
**23.01.85 Bulletin 85/04**

(84) Designated Contracting States:
**AT BE CH DE FR GB IT LI LU NL SE**

(56) References cited:

**CHEMICAL ABSTRACTS, vol. 75, no. 23, 6th December 1971, page 279, column 1, no. 140287x, Columbus, Ohio, USA**

(73) Proprietor: **FINE ORGANICS LIMITED**
**Erimus House Queen's Square**
**Middlesbrough Cleveland, TS2 1AA (GB)**

(72) Inventor: **Hollowood, John**
**Beech Croft**
**Nawton, York YO6 5TU (GB)**

(74) Representative: **Harrison, Michael Robert et al**
**URQUHART-DYKES & LORD 11th Floor Tower House Merrion Way**
**Leeds LS2 8PB West Yorkshire (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 060 049 B1**

**Description**

This invention relates to processes for the production of intermediates useful in the preparation of cyanoguanidines. Cyanoguanidines are useful, for instance, as anti-ulcer drugs, examples being cimetidine and etintidine. Specifically the present invention is concerned with a process for the preparation of dialkyl cyanodithioimino carbonates, such a process being suitable at plant scale in the commercial production of intermediates for conversion to cyanoguanidines used as pharmaceutical compounds.

A known commercial method for the preparation of dimethyl cyanodithioimino carbonate involves the addition of an alcoholic solution of potassium hydroxide to a solution of cyanamide in an alcohol and carbon disulphide. The temperature is kept below 15°C and the product is the dipotassium salt of cyanodithioimino carbonate. The isolated dipotassium salt is charged to water and dimethyl sulphate added to the solution, again maintaining the temperature below 15°C. The product is dimethyl cyanodithioimino carbonate at an overall yield of about 66%.

The above-described process requires the use of a mixed alcoholic solvent system in order to obtain a good yield of the dipotassium salt. Accordingly, recovery of the alcohols is difficult and makes the process an expensive one.

The dipotassium salt must be isolated and since the material is highly caustic it requires special handling.

The process requires the use of potassium hydroxide which is an expensive reagent.

The use of alcohols in the preparation of the dipotassium salt leads to the formation of xanthates as by-products and thereby reduces the yield which can be obtained.

According to the present invention there is provided a process for preparing a dialkyl cyanodithioimino carbonate as an intermediate in the commercial production of a cyanoguanidine for pharmaceutical use, said dialkyl cyanodithioimino carbonate having the formula

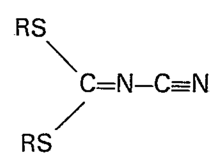

(I)

where each R is independently an alkyl group, the process comprising contacting together, under reaction conditions alkali, cyanamide, a sulphur providing agent and an alkylating agent, the process being conducted in the absence of any substantial amount of solvent other than water.

Preferably R is lower alkyl (preferably $C_1$—$C_4$ alkyl) and more preferably is methyl.

The alkali may be sodium hydroxide and indeed it is an advantage of the process of the invention that sodium hydroxide may be used rather than the more expensive potassium hydroxide.

The process of the invention may be carried out without using an organic solvent and, therefore, is essentially much more simple and much less expensive than a process in which a solvent is involved, even if it is possible to recover and recycle the solvent. Furthermore, the avoidance of large quantities of organic solvent means that the batch yield (the yield based on the total volume of the batch) may be much higher. Furthermore, there is no need for expensive equipment in order to separate off, for instance, by distillation volatile solvents.

Preferably the alkylating agent is dialkyl sulphate and more preferably is dimethyl sulphate.

Preferably the sulphur providing agent is carbon disulphide.

Preferably the process is conducted in the presence of surface active agent. The surface active agent may be non-ionic, cationic or anionic wetting agent, an example being the non-ionic wetting agent ethoxylated nonyl phenol. The surface active agent may be a phase transfer catalyst such as benzyltrimethylammonium chloride.

The reaction may be carried out at a temperature higher than 15°C, thereby allowing advantage to be taken of an increased reaction rate at increased temperature.

The process can be carried out as a "one pot" reaction without the need to isolate the intermediate salt (for instance, the dipotassium salt or disodium salt).

A preferred process for producing a dialkyl cyanodithioimino carbonate of the formula I as given above comprises contacting together cyanamide, carbon disulphide and, if desired a surface active agent, adding alkali and then an alkylating agent. Preferably the reaction mixture is agitated after the addition of alkali and prior to the addition of alkylating agent until the mixture becomes homogeneous. Preferably the alkylating agent is added at a rate such that the temperature does not rise above about 15°C. More preferably the rate of addition is such that the temperature is maintained between 10 and 15°C.

The order of addition of reactants may be varied and the comparative proportions in which the reactants may be mixed together may also be varied. By way of example, alkali may be added to cyanamide followed by addition of the carbon disulphide. In an alternative embodiment, the cyanamide may be first mixed with the carbon disulphide and alkali subsequently added. It is, however, important to ensure as far as possible that undesirable by-products are not formed, and in this respect an unacceptable product is obtained if oils are formed during the process. It is preferred that the first stage of the process, namely, the formation of the anion, prior to the addition of

alkylating agent, be carried out in such a way that the cyanamide and alkali are in contact for the shortest possible time. In the case where alkali is being added to a mixture of cyanamide and carbon disulphide, the alkali should be added as quickly as possible, the temperature of the reaction mixture being allowed to rise, as a result of the fast alkali addition, to a comparatively high temperature, the limit being the boiling point of the carbon disulphide (44°C). It has been surprisingly discovered that very high yields of the desired product can be obtained, without significant amounts of undesirable by-products, as a result of this comparatively high temperature reaction as long as the alkali is added over a short period. A preferred short period is up to one and a half hours but instead of adding the alkali to a mixture of cyanamide and carbon disulphide it is possible to achieve the same result, that is avoidance of a prolonged period of contact between the cyanamide and alkali, by adding to the $CS_2$ simultaneously both cyanamide and alkali. In this case the reactants can be added at any rate as long as they only come into contact in the presence of the carbon disulphide. Again the temperature may be allowed to rise up to a temperature below that of the boiling point of carbon disulphide in order to achieve a fast reaction rate. Fast addition of the reactants will in itself cause the reaction temperature to rise.

Examples of processes in accordance with the present invention will now be described.

Example I

Sodium hydroxide (40 g.) was dissolved in 230 ml. water and added to 50% aqueous cyanamide (42 g.) keeping the temperature between 0—10°C. 1 g. of benzyltrimethylammonium chloride (a phase transfer catalyst) was added prior to the addition of the carbon disulphide (34.2 g.). The solution was then warmed to 30°C and stirred until it was homogeneous, i.e. all the carbon disulphide had reacted.

To the resulting solution dimethyl sulphate (14.0 g.) was added keeping the temperature between 20—25°C. This was stirred at one hour before heating to 30°C. On cooling back to 15°C the precipitated product was filtered off and dried to give 65 g. of the dimethyl cyanodithioimino carbonate (90% yield).

Example II

To a 50% aqueous solution of cyanamide (84 g.; 1 mole), there are added 200 ml water and carbon disulphide (68.4 g.; 0.9 mole) together with 1 g. of a phase transfer catalyst (benzyltrimethylammonium chloride). The mixture is stirred and heated to 25°C. Aqueous sodium hydroxide solution (80. g; 2 moles, dissolved in 240 ml water) is then added over a period of 1.5 hours, controlling the temperature between 28°C and 30°C. The mixture is then stirred at this temperature for a further 6 hours giving a clear orange-red solution. Dimethyl sulphate (240 g.; 1.9 moles) is then added over a period of 1 hour maintaining the temperature between 20 and 25°C. When the addition is complete the reaction is stirred for one hour before warming to 35°C to destroy an excess dimethyl sulphate. On cooling back to 12 to 15°C the product is filtered and washed with water (two washings of 100 ml each). The product is dried under vacuum to give 120.5 g. (82.5% of theory based on cyanamide charge).

It has been found that particularly good quality products may be obtained in high yield by conducting the reaction in accordance with the following conditions.

On the basis of use of 1 mole of cyanamide (84 g, typically in a 50% aqueous solution), there should be added from 150 to 500 ml water and from 0.8 to 1.0 mole carbon disulphide together with an amount of phase transfer catalyst up to 5 g. The mixture should be stirred at a temperature in the range 0 to 30°C. Aqueous sodium hydroxide solution (from 1.8 to 2.4 moles dissolved in from 200 to 300 ml water) is then added over a period of from 30 minutes to 12 hours (preferably less than 2 hours and more preferably a period of from 1 to $1\frac{1}{2}$ hours) controlling the temperature between 20 and 40°C (or up to the boiling point of carbon disulphide at 44°C). The mixture is then stirred until a homogeneous solution is obtained (indicating that all or substantially all the carbon disulphide has reacted), typically taking from 6 to 10 hours. The mixture is then cooled to about 0°C and from 1.6 to 2.2 moles of dimethyl sulphate are added, the rate of addition being such that the temperature is allowed to rise to from 10 to 15°C. The addition may typically take from 1 to 5 hours. After completion of the addition the mixture is stirred for a further period, typically about 4 hours. The product is then filtered and washed with water, typically with two washings of 100 ml each.

An example of a reaction conducted in accordance with the above-mentioned conditions will now be described.

A process similar to that described in this Example has been conducted and found to yield excellent quality products on a larger scale. To a 500 gallon vessel, 190 Kg of 50% aqueous cyanamide, 450 Kg water and 155 Kg carbon disulphide were added and the mixture warmed with stirring to 25°C. 724 Kg of 25% aqueous sodium hydroxide was added over a period from 1 to 1.5 hours, keeping the temperature within the range 28 to 30°C by control of the precise rate of addition. The reaction mixture was then stirred at this temperature until it was homogeneous, the period being approximately six hours. The reaction was then cooled and 543 Kg of dimethyl sulphate was added keeping the temperature between 20 and 25°C. When the addition was complete the reaction mixture was stirred for one hour at 20 to 25°C and then heated to 35 to 40°C to destroy excess dimethyl sulphate. The contents of the vessel were cooled to 12 to 15°C and the product centrifuged, the product being washed while on the centrifuge.

Example III

To a 50% aqueous solution of cyanamide (84 g; 1 mole) there are added 200 ml of water and carbon disulphide (68.4 g; 0.9 mole) together with 1 g benzyltrimethylammonium chloride. The mixture is stirred and heated to 20°C. Aqueous sodium hydroxide solution (80 g; 2 moles dissolved in 240 ml water) is then added over a period of $1\frac{1}{2}$ hours, controlling the temperature to about 35°C. The mixture is then stirred at this temperature until a homogeneous solution is obtained, this taking about 6 hours. The mixture is then cooled to 0°C and then dimethyl sulphate (240 g; 1.9 moles) is added. The addition is at a rate such that the temperature is allowed to rise to from 10 to 15°C. The addition of dimethyl sulphate takes about 1 hour. After completion of the addition of dimethyl sulphate the mixture is stirred for about 4 hours. The mixture is then filtered and the filtered product is then washed with water (two washings of 100 ml each). The product is dried under vacuum and the yield is 131 g (90% of theory based on cyanamide charge).

The above-described Example III was repeated but without the addition of benzyltrimethylammonium chloride, or indeed any other surface active agent. In this case 110 g of dimethyl cyanodithioimino carbonate were obtained (75% of theory based on cyanamide charge).

## Claims

1. A process for preparing a dialkyl cyanodithioimino carbonate as an intermediate in the commercial production of a cyanoguanidine for pharmaceutical use said dialkyl cyanodithioimino carbonate having the formula

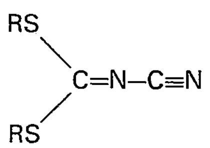

I

where each R is independently an alkyl group, characterised in that the process comprises contacting together, under reaction conditions, alkali, cyanamide, a sulphur providing agent and an alkylating agent, the process being conducted in the absence of any substantial amount of solvent other than water.

2. A process according to claim 1 characterised in that the reactants are contacted together in such a way as to avoid prolonged contact of cyanamide and alkali.

3. A process according to claim 1 or claim 2 characterised in that the alkali is added to a mixture of carbon disulphide and cyanamide over a period of up to 2 hours to form the cyanodithioimino carbonate anion, said anion being contacted with alkylating agent to form the dialkyl cyanodithioimino carbonate of the formula I.

4. A process according to claim 1 or claim 2 characterised in that the alkali and cyanamide are added separately and simultaneously to carbon disulphide in order to form the cyanodithioimino carbonate anion and the anion is contacted with alkylating agent to form the dialkyl cyanodithioimino carbonate of the formula I.

5. A process according to any of the preceding claims characterised in that the process is conducted in the presence of a surface active agent.

6. A process according to any of the preceding claims characterised in that R is a lower alkyl.

7. A process according to claim 6 characterised in that R is methyl.

8. A process according to any of the preceding claims characterised in that the alkali is sodium hydroxide or potassium hydroxide.

9. A process according to any of the preceding claims characterised in that the alkylating agent is a dialkyl sulphate.

10. A process according to any of the preceding claims characterised in that the alkylating agent is dimethyl sulphate.

11. A process according to any of the preceding claims characterised in that the sulphur providing agent is carbon disulphide.

## Patentansprüche

1. Verfahren zur Herstellung eines Dialkylcyanodithioiminocarbonats als ein Zwischenprodukt für die großtechnische Herstellung eines Cyanoguanidins zur pharmazeutischen Anwendung, wobei dieses Dialkylcyanodithioiminocarbonat die folgende Formel hat

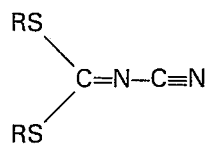

I

worin R jeweils unabhängig une Alkylgruppe ist, dadurch gekennzeichnet, daß man ein Alkali, Cyanamid, ein für Schwefel sorgendes Mittel und ein Alkylierungsmittel unter Reaktionsbedingungen in Kontakt hält und das Verfahren in Abwesenheit irgendeiner wesentlichen Menge an Lösungsmittel mit Ausnahme von Wasser durchführt.

2. Verfahren nach Anspruch 1, dadurch gekennzeichnet, daß man die Reaktanten so in Kontakt hält, daß ein längerer Kontakt zwischen Cyanamid und dem Alkali vermieden wird.

3. Verfahren nach Anspruch 1 oder Anspruch 2, dadurch gekennzeichnet, daß man das Alkali

während einer Zeitdauer von bis zu 2 Stunden zu einem Gemisch aus Kohlenstoffdisulfid und Cyanamid gibt und das so gebildete Cyanodithioiminocarbonatanion mit dem Alkylierungsmittel in Kontakt bringt und so das Dialkylcyanodithioiminocarbonat der Formel I bildet.

4. Verfahren nach Anspruch 1 oder Anspruch 2, dadurch gekennzeichnet, daß man das Alkali und das Cyanamid getrennt und zegleich mit dem Kohlenstoffdisulfid zusetzt und das so gebildete Cyanodithioiminocarbonatanion mit dem Alkylierungsmittel in Kontakt bringt und so das Dialkylcyanodithioiminocarbonat der Formel I bildet.

5. Verfahren nach einem der vorhergehenden Ansprüche, dadurch gekennzeichnet, daß man das Verfahren in Gegenwart eines oberflächenaktiven Mittels durchführt.

6. Verfahren nach einem der vorhergehenden Ansprüche, dadurch gekennzeichnet, daß R für Niederalkyl steht.

7. Verfahren nach Anspruch 6, dadurch gekennzeichnet, daß R Methyl ist.

8. Verfahren nach einem der vorhergehenden Ansprüche, dadurch gekennzeichnet, daß das Alkali Natriumhydroxid oder Kaliumhydroxid ist.

9. Verfahren nach einem der vorhergehenden Ansprüche, dadurch gekennzeichnet, daß das Alkylierungsmittel ein Dialkylsulfat ist.

10. Verfahren nach einem der vorhergehenden Ansprüche, dadurch gekennzeichnet, daß das Alkylierungsmittel Dimethylsulfat ist.

11. Verfahren nach einem der vorhergehenden Ansprüche, dadurch gekennzeichnet, daß das für Schwefel sorgende Mittel Kohlenstoffdisulfid ist.

**Revendications**

1. Un procédé pour la préparation d'un cyanodithioiminocarbonate de dialkyle comme intermédiaire dans la production industrielle d'une cyanoguanidine à usage pharmaceutique, ledit cyanodithioiminocarbonate de dialkyle répondant à la formule

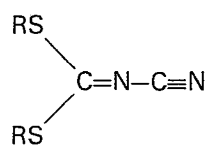

I

dans laquelle chaque R est indépendamment un groupe alkyle, caractérisé en ce que le procédé comprend le contact mutuel dans des conditions réactionnelles, d'un alcali, de cyanamide, d'un agent apportant du soufre et d'un agent d'alkylation, le procédé étant effectué en l'absence de toute quantité notable de solvant autre que l'eau.

2. Un procédé selon la revendication 1, caractérisé en ce que les composés réagissants sont mis en contact mutuel de façon à éviter un contact prolongé du cyanamide et de l'alcali.

3. Un procédé selon la revendication 1 ou la revendication 2, caractérisé en ce que l'alcali est ajouté à un mélange de disulfure de carbone et de cyanamide en une période d'au plus 2 heures, pour former l'anion cyanodithioiminocarbonate, ledit anion étant mis en contact avec un agent d'alkylation pour former le cyanodithioiminocarbonate di dialkyle de formule I.

4. Un procédé selon la revendication 1 ou la revendication 2, caractérisé en ce que l'alcali et le cyanamide sont ajoutés séparément et simultanément au disulfure de carbone pour former l'anion cyanodithioiminocarbonate et l'anion est mis en contact avec un agent d'alkylation pour former le cyanodithioiminocarbonate de dialkyle de formule I.

5. Un procédé selon l'une quelconque des revendications précédentes, caractérisé en ce que le procédé est effectué en présence d'un agent tensioactif.

6. Un procédé selon l'une quelconque des revendications précédentes, caractérisé en ce que R est un alkyle inférieur.

7. Un procédé selon la revendication 6, caractérisé en ce que R est un méthyle.

8. Un procédé selon l'une quelconque des revendications précédentes, caractérisé en ce que l'alcali est l'hydroxyde de sodium ou l'hydroxyde de potassium.

9. Un procédé selon l'une quelconque des revendications précédentes, caractérisé en ce que l'agent d'alkylation est un sulfate de dialkyle.

10. Un procédé selon l'une quelconque des revendications précédentes, caractérisé en ce que l'agent d'alkylation est le sulfate de diméthyle.

11. Un procédé selon l'une quelconque des revendications précédentes, caractérisé en ce que l'agent apportant du soufre est le disulfure de carbone.